# EUROPEAN PATENT SPECIFICATION

(11) **EP 3 359 965 B1**
(45) Date of publication and mention of the grant of the patent: **29.06.2022**
(21) Application number: 16778350.5
(22) Date of filing: 06.10.2016
(51) Int. Cl.: G01N 33/68

(54) **METHODS FOR CARDIOVASCULAR CONDITIONS**
METHODEN FÜR KARDIOVASKULARE ERKRANKUNGEN
METHODES POUR LES MALADIES CARDIOVASCULAIRES

(30) Priority: 07.10.2015 GB 201517731
(43) Date of publication of application: 15.08.2018
(73) Proprietor: Randox Laboratories Ltd., Crumlin, County Antrim BT29 4QY (GB)
(72) Inventor: MCCENEANEY, David, Portadown Armagh BT66 5QQ (GB)
(86) International application number: PCT/EP2016/073892
(87) International publication number: WO 2017/060359

(56) References cited:
- EP-A1- 2 687 853
- WO-A2-2010/144553
- OKAMOTO FUMIO ET AL: "HUMAN HEART-TYPE CYTOPLASMIC FATTY ACID-BINDING PROTEIN (H-FABP) FOR THE DIAGNOSIS OF ACUTE MYOCARDIAL INFARCTION. CLINICAL EVALUATION OF H-FABP IN COMPARISON WITH MYOGLOBIN AND CREATINE KINASE ISOENZYME MB", CLINICAL CHEMISTRY AND LABORATORY MEDICINE, DE GRUYTER, DE, vol. 38, no. 3, 1 March 2000 (2000-03-01), pages 231-238, XP008077883, ISSN: 1434-6621, DOI: 10.1515/CCLM.2000.034
- JAN F.C GLATZ ET AL: "Fatty acid-binding protein and the early detection of acute myocardial infarction", CLINICA CHIMICA ACTA, vol. 272, no. 1, 1 April 1998 (1998-04-01) , pages 87-92, XP055341905, AMSTERDAM, NL ISSN: 0009-8981, DOI: 10.1016/S0009-8981(97)00255-6
- SEINO YOSHIHIKO ET AL: "Use of a whole blood rapid panel test for heart-type fatty acid-binding protein in patients with acute chest pain: Comparison with rapid troponin T and myoglobin tests", AMERICAN JOURNAL OF MEDICINE, EXCERPTA MEDICA, INC, UNITED STATES, vol. 115, no. 3, 15 August 2003 (2003-08-15), pages 185-190, XP002454291, ISSN: 0002-9343, DOI: 10.1016/S0002-9343(03)00325-5
- Y SEINO ET AL: "Office Cardiologists Cooperative Study on Whole Blood Rapid Panel Tests in Patients With Suspicious Acute Myocardial Infarction", CIRCULATION JOURNAL, vol. 68, no. 68, 1 February 2004 (2004-02-01), pages 144-148, XP055341911,
- RAMASAMY I ED - MUSSAP MICHELE ET AL: "Biochemical markers in acute coronary syndrome", CLINICA CHIMICA ACTA, ELSEVIER BV, AMSTERDAM, NL, vol. 412, no. 15, 3 April 2011 (2011-04-03), pages 1279-1296, XP028218471, ISSN: 0009-8981, DOI: 10.1016/J.CCA.2011.04.003 [retrieved on 2011-04-08]
- WUNDERLICH M T ET AL: "Release of brain-type and heart-type fatty acid-binding proteins in serum after acute ischaemic stroke", JOURNAL OF NEUROLOGY, STEINKOPFF-VERLAG, DA, vol. 252, no. 6, 1 June 2005 (2005-06-01), pages 718-724, XP019342470, ISSN: 1432-1459

## Description

### BACKGROUND

Chest pain places a heavy burden on the healthcare system. Around 6% of all people attending the emergency department (ED) in England and Wales (-700,000 individuals) report chest pain as their primary complaint. Around two thirds of those patients are admitted to hospital for further investigation and make up over a quarter of all acute medical admissions. Despite the high admission rate, only around a quarter of admitted patients are diagnosed as acute coronary syndrome or "ACS" (ACS includes ST elevation myocardial infarction or STEMI, non ST elevation myocardial infarction or NSTEMI and unstable angina). The current methodology for testing for ACS is the electrocardiogram (ECG) which will identify STEMI; expeditied percutaneous intervention (PCI, insertion of coronary stent) is the current standard of care. Elevated cardiac biomarkers (in particular Troponin) in the presence of a normal ECG or one with nonspecific ECG changes identifies NSTEMI. The current diagnostic paradigm requires admission and observation over a period of several hours with sequential cardiac biomarker measurements. This implies an overcautious approach (low specificity), and that many patients who don't have the condition are admitted to prevent false negatives. Conversely, if the relatively small proportion of patients who are discharged from the ED were to be called back and re-examined (troponin tested at the appropriate time), it would show that up to 6% of patients had an unidentified acute myocardial infarction - the mortality rate for these patients is at least twice that of those who are admitted.

Angina, heart attack and stroke, may be caused by the same problem, atherosclerosis, a condition in which arteries become narrowed by a gradual build-up of fatty material or 'atheroma' within their walls. Over time arteries may become so narrow that they cannot deliver enough oxygen-rich blood to the heart resulting in angina. If a piece of the atheroma breaks away it may cause a blood clot blocking the coronary artery or arteries leading to the brain, causing a heart attack or stroke, respectively. Risk factors of these cardiovascular conditions (angina, heart attack, stroke and atherosclerosis) include smoking, high blood pressure, high blood cholesterol, physical inactivity, obesity, diabetes, family history of heart disease and ethnic background. Diagnosing ACS and atherosclerosis thus presents a challenge.

The simplest and cheapest diagnostic aid is the 12 lead electrocardiogram. Changes that may indicate ischaemia or infarction include ST segment elevation or depression, left bundle branch block (LBBB), T wave changes and ultimately pathological Q waves. The initial ECG, however, only has a sensitivity of 30% for diagnosing myocardial infarction (MI). The use of multi-lead surface mapping ECG increases sensitivity modestly, but still without sufficient negative predictive value to rule out MI. Blood-based biomarker assays are also used for diagnosis. Currently cardiac troponin measurement (4th generation cardiac troponin T and cardiac troponin I) remains the gold standard biomarker assay of myocardial necrosis. The release kinetics of troponin after myocardial injury and subsequent necrosis means that current sampling and measurement protocol involves obtaining two measurements at T=0 and T= ≥6 hours. Conventionally in the UK a 12 hour post chest pain sample is considered acceptable. The development of highly sensitive troponin (hsTn) assays has improved the diagnostic accuracy of troponin measurements at presentation in comparison to standard troponin assays. As a significant proportion of patients will present within 6 hours of symptoms occurring, there has been extensive research interest in early biomarkers of myocardial necrosis. Heart fatty acid binding protein (H-FABP) has been prospectively investigated in this regard. H-FABP is a small protein (14-15 kDa) released from cardiac myocytes. Detectable levels occur within 3 hours after myocardial injury and return to baseline within 12-24 hours. EP2687853 and EP2846285 report the use of troponin, h-FABP and ECG, for the diagnosis of AMI. A study of Wunderlich et al. (J. Neurol. (2005) 252:718-724) aimed at an analysis of the release of Braintype and Heart-type Fatty Acid- Binding Proteins (B-FABP and HFABP) in acute ischaemic stroke and their potential value as neurobiochemical markers of brain damage.

Improvements in diagnostic assays and methodologies for patients presenting with chest pain in relation to acute myocardial infarction detection are desirable and ongoing but assays which also incorporate methods and improvements relating to the identification of other cardiovascular conditions such as atherosclerosis and stroke are also needed. An improvement in the scope of cardiovascular conditions detected in patients with chest pain at the time of presentation would optimise patient care, save lives and afford long-term cost savings.

### Figures

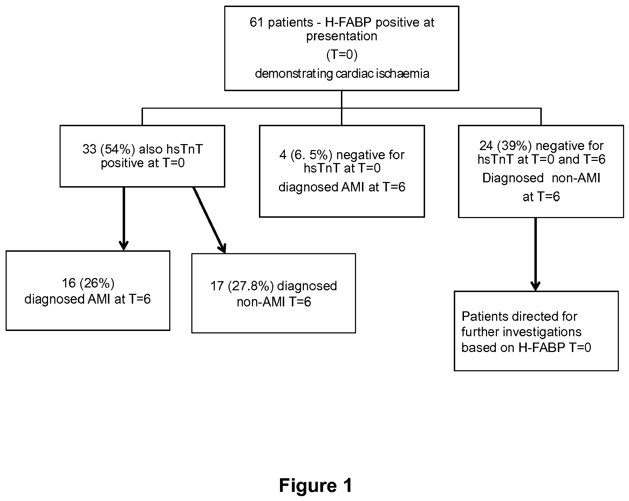

Figure 1 Schematic of Results from Study

### SUMMARY OF THE INVENTION

The invention describes a method that supports the determination of a cardiovascular conditions being atherosclerosis, ischaemic stroke and deep vein thrombosis in an individual with chest pain. The method uses the measurement of H-FABP in addition to the use of ECG and the measurement of cardiac troponin.

### DETAILED DESCRIPTION OF THE INVENTION

The invention relates to a method of determining a possible cardiovascular condition in an individual with chest pain comprising determining H-FABP in an ex vivo sample taken from the individual who has a negative electrocardiogram and negative cardiac troponin and based on the determination of H-FABP categorising the individual as healthy or having a possible cardiovascular condition characterised in that a positive H-FABP determination indicates that the possible cardiovascular condition is atherosclerosis, ischemic stroke or deep vein thrombosis. Further embodiments are defined in the appended dependent claims. The disclosure describes a method of determining a possible cardiovascular condition in an individual with chest pain comprising determining H-FABP in an ex *vivo* sample taken from the individual and based on the determination of H-FABP categorising the individual as healthy or having a possible cardiovascular condition. The cardiovascular condition is preferably acute myocardial infarction, atherosclerosis or thrombosis. Preferably, the thrombosis is ischaemic stroke or deep vein thrombosis (DVT).

The disclosure further describes a method of determining a possible cardiovascular condition in an individual with chest pain comprising determining H-FABP in an ex *vivo* sample taken from the individual and subjecting the individual to an electrocardiogram and based on the determination of H-FABP and the electrocardiogram results and an optional further step comprising determination of cardiac troponin, categorising the individual as healthy or having a possible cardiovascular condition. 'Determination' implies the level or concentration is measured or a cut-off value is exceeded. The method can be applied with or without electrocardiography, although implementation is likely with an ECG as this is current standard clinical practice. The cardiovascular condition determined can be any disease of the heart and circulation but the method is most effective at supporting the determination of acute myocardial infarction, atherosclerosis and thrombosis.

According to the invention, the cardioavascular condition is atherosclerosis, ischemic stroke or DVT.

is ischaemic stroke or DVT. As described herein, a positive H-FABP determination and a positive electrocardiogram categorises the individual as possible acute myocardial infarction; a positive H-FABP determination, a negative electrocardiogram and negative cardiac troponin categorises the individual as possible atherosclerosis or stroke; and a positive H-FABP determination, a negative electrocardiogram and positive cardiac troponin categorises the individual as possible acute myocardial infarction. According to the invention, a positive H-FABP determination, a negative electrocardiogram and negative cardiac troponin categorises the individual with chest pain as possible atherosclerosis, DVT or ischaemic stroke. For all the methods the assessment of whether H-FABP is positive in a patient is based on the comparison of data from a healthy individual or a group of healthy individuals (the reference level) and whether the patient has a measured level of H-FABP which is higher than a healthy individual or higher than a recognised value from a group of healthy individuals. If the comparison is based on a healthy individual it is preferable that this measurement is taken from the individual with chest pain when disease free. The group value can be, for example, a mean, a median or a percentile measurement (the 95^{th} or 99^{th} percentile are preferred). In a preferred embodiment of the invention the method requires that an individual is positive for H-FABP when the measured value is greater than the 95^{th} percentile of the upper reference level. In a further example, the method requires that an individual is positive for H-FABP when the measured value is greater than the 99^{th} percentile of the upper reference level. A further example, the method requires that an individual is positive for H-FABP when the measured value is greater than about 2.50 ng/ml, greater than about 3.00 ng/ml, or greater than about 3.50 ng/ml. The comparison data can be based on stratified cohorts, such stratification being, for example, age, gender, smoker/non-smoker, BMI. Furthermore, the comparison data derived from a control group could evolve slightly over time as and if more data is added. In a preferred embodiment the method involves the measurement of H-FABP within one hour of admission to or presentation at a medical facility. In a further example, the method involves the measurement of H-FABP within one hour of the onset of chest pain in the individual.

The disclosure also describes a method of assessing whether a patient with chest pain requires drug therapy or a surgical procedure comprising determining H-FABP in an ex *vivo* sample taken from the individual and subjecting the individual to an electrocardiogram and based on the determination of H-FABP and the electrocardiogram results and an optional further step comprising determination of cardiac troponin, categorising the individual as healthy or having a possible cardiovascular condition, and subjecting the patient with a possible cardiovascular condition to a further procedure and/or testing the result of which may be followed by the patient being prescribed drugs or being recommended for a surgical procedure. The surgical procedure recommended can be any procedure which alleviates or manages the cardiovascular condition such as the administration of a stent or heart surgery and the prescribed drug can be any drug which alleviates or manages the cardiovascular condition such as thrombolytics, anti-atherosclerosis drugs etc. As with the previous methods if the determined H-FABP is higher than a healthy individual or higher than a recognised value from a group of healthy individuals i.e. upper 95^{th} or 99^{th} percentile or greater then about 2.50 ng/ml, greater than about 3.00 ng/ml, or greater than about 3.50 ng/ml, the further procedure and/or testing is effected as soon as possible, preferably within 3 months, more preferably within 1 month, most preferably within 1 week of obtaining the H-FABP positive determination; ideally the further procedure/testing is done as soon as possible following knowledge of a positive H-FABP determination i.e. on the same day. However, it is recognised that such an immediate response is not always practical or achievable within a medical facility. Common surgical procedures which might be recommended following the further testing are coronary artery bypass grafting, stent insertion and the prescription of anti-atherosclerosis drugs and/or thrombolytics.

The disclosure further describes a method of treatment for atherosclerosis comprising:
i. measurement of a patient ECG and measurement of the H-FABP and cardiac troponin in a sample taken from a patient, the patient having suffered or who is suffering from chest pain, the troponin and ECG measurement having been found negative, and the H-FABP measurement having been found positive;
ii. subjecting the patient to further tests to confirm the presence and degree of atherosclerosis
iii. based upon ii., prescribing the individual anti-atherosclerosis drugs and/or subjecting the patient to a surgical intervention.

The disclosure also describes a method of treatment of stroke comprising:
i. measurement of a patient ECG and measurement of the H-FABP and cardiac troponin in a sample taken from a patient, the patient having suffered or who is suffering from chest pain, the troponin and ECG measurement having been found negative, and the H-FABP measurement having been found positive;
ii. subjecting the patient to further tests to confirm the presence and degree of stroke
iii. based upon ii., prescribing the individual anti-stroke drugs and/or subjecting the patient to a surgical intervention.

The disclosure also describes a method of treatment of thrombosis especially ischaemic stroke or DVT comprising:
i. measurement of a patient ECG and measurement of the H-FABP and cardiac troponin in a sample taken from a patient, the patient having suffered or who is suffering from chest pain, the troponin and ECG measurement having been found negative, and the H-FABP measurement having been found positive;
ii. subjecting the patient to further tests to confirm the presence of thrombosis
iii. based upon ii., prescribing the individual anti-thrombolytic drugs and/or subjecting the patient to a surgical intervention.

Drug and surgical interventions for atherosclerosis and thrombosis for all the methods include where applicable stents, by-pass surgery, anti-atherosclerosis drugs, anti-coagulation drugs, thrombolytic drugs (enzyme treatment), mechanical thrombectomy, leg-stockings, inferior vena cava filters, surgical pressure release. The sample from the individuals in the methods can be selected from a body fluid, separated cells or tissue sample. Most preferably the sample is a body fluid, even more preferably the sample is blood, serum or plasma; serum is especially preferred. In an example of the current invention the ex *vivo* determinations of H-FABP and cardiac troponin are carried out by immunoassay using any means known in the art. In another example the immunoassay is incorporated into an automated analyser device.

In the disclosure the following definitions apply: "Possible" in the context of a diagnostic test or procedure implies the resultant diagnostic measurement supports or lends weight to a particular diagnosis. "Troponin" implies one or more of troponin T, troponin I and high sensitivity troponin unless referred to by its specific sub-type i.e. TnT (troponin T), Tnl (troponin I), hsTn (high sensitivity troponin)"lndividual" and "patient" are interchangeable unless the context suggests otherwise.

"Medical facility" includes hospital, emergency department, clinic, doctor's surgery, ambulance, medical vehicle, portable or non-permanent medical structure incorporating the required medical equipment to undertake the methods.

"Cardiovascular Condition" implies a cardiovascular-related (heart and circulation) disease or event that has occurred, has been occurring over a prolonged period, or has recently occurred and is on-going.

Acute coronary syndrome (ACS) refers to a set of signs and symptoms usually a combination of chest pain and other features interpreted as being a result of abruptly decreased blood flow to the heart (cardiac ischaemia). The subtypes of ACS include unstable angina (UA, not usually associated with heart muscle damage) and two forms of myocardial infarction (non-ST & ST elevated) in which heart muscle is damaged.

Acute myocardial infarction (AMI or MI), more commonly known as a heart attack, refers to a medical condition that occurs when the blood supply to a part of the heart is interrupted, most commonly due to rupture of a vulnerable plaque. The resulting ischaemia (oxygen shortage) if left untreated for a sufficient period can cause damage and/or death to heart tissue.

The term 'H-FABP' as used herein refers to heart fatty acid-binding protein, also known as fatty acid binding protein 3 (FABP-3) and fragments or partial peptides thereof, which in humans is encoded on the *FABP3* gene. It will be understood to those skilled in the art that H-FABP incorporates any variant polypeptides which share the same essential biological and immunological properties as the specific

H-FABP peptides used in the current invention. H-FABP is an unbound, low molecular weight protein (~15kDa), located primarily in the cytoplasm of cardiac myocytes and is involved in the intracellular uptake of long chain fatty acids in the myocardium.

The terms 'troponin' (Tn) and 'cardiac troponin' (cTn) as used by the current disclosure refer to all isoforms of troponin which are expressed in the cells of the heart, preferably they refer to cardiac troponin T or cardiac troponin I and fragments or partial peptides thereof, most preferably they refer to cardiac troponin T (cTnT).

The 'reference values' or 'cut-off levels' used in the current invention are commonly acceptable levels of cTnT which are indicative of acute myocardial infarction. For the purposes of the current disclosure any value less than or within the range of these reference levels may be referred to as a 'negative' test, while any value greater than these reference levels may be referred to as a 'positive' test. Preferably for cTnT the reference value is based upon the 99th percentile of a reference population as determined by the manufacturer. In the current disclosure the preferred cut-off level used for cTnT was 0.014ng/ml and any cTnT value below this reference value indicated that a recent AMI has not occurred. Those skilled in the art will understand that the cut-off level can vary depending on the manufacturer of the kit used to detect a biomarker. Therefore any cut-off levels recommended in a high sensitivity troponin T assay can fall under the scope of the current disclosure. While cTnT is the preferred cardiac troponin of the invention, any cardiac troponin which could be an indicator of tissue damage may also be used such as cardiac troponin I. Wherein the cardiac troponin is cardiac troponin I (cTnl) the preferred cut-off value for ruling out a myocardial infraction is based on the 99th percentile of a reference population as determined by the assay manufacturer, and a cTnl value less than this reference value is considered a negative result and indicates that a recent AMI has not occurred. Preferably for H-FABP the reference value is based on the 95th percentile of a reference population. Any H-FABP value below this value is considered a negative result and indicates that a recent AMI has not occurred. Those skilled in the art will recognise that alternative H-FABP cut-off values recommended for ruling out acute myocardial infarction could also be used. Alternatively, for H-FABP the reference value is based on the 99th percentile of a reference population.

In the context of the current invention the phrase 'positive' or 'abnormal' ECG refers to any ECG reading which has ECG features or characteristics which are commonly associated with an acute myocardial infarction. These may include for example ST segment elevation or new onset left bundle branch block or ST segment depression or T wave inversion. A 'negative' or 'normal' ECG as referred to herein indicates an ECG reading devoid of such features or characteristics listed above. The ECG is preferably a standard 12-lead ECG but any suitable variation known to those skilled in the art may be used to interpret the electrical activity of the heart. The ECG may be a paramedic ECG taken en route to the hospital or medical facility or an emergency department ECG.

The 'subject', 'patient' or 'individual' of the current invention presents to a medical facility (preferably an Emergency Department, doctor's surgery or ambulance) with chest pain. Cardiovascular conditions according to the invention are ischaemic stroke, atherosclerosis and DVT.

### Methods

A 7 month study was conducted at Craigavon Area Hospital Northern Ireland. Patient blood samples were collected upon arrival to Accident and Emergency and then again at 6 hours; Troponin T was measured in these samples in accordance with the hospital protocol as well as H-FABP (first sample only). Patients were subject to electrocardiography.

A total of 69 patients, all ECG negative, were involved consisting of 24 females and 45 males with a mean age of 66.8 years. Patients were excluded if they didn't have samples taken at both time points; therefore, 62 patients were included in the final analysis. One of the 62 patients was negative for both troponin and H-FABP demonstrating that this patient's chest pain was not of cardiac origin.

**Table 1 The following criteria was used for diagnosis**

| **T=0 hour** | **Concentration** | **Result** | **Decision** |
|---|---|---|---|
| hsTnT | <14ng/l | negative | Perform 6 hour hsTnT |
| hsTnT | ≥14ng/l | Positive | Perform 6 hour hsTnT |
| H-FABP | 95^{th} percentile | Negative | |
| H-FABP | 95^{th} percentile | Positive | |
| | | | |
| T=6 hour | | | |
| hsTnT | ≥7ng/l delta change between T=0 and T=6 reading | Positive | |
| hsTnT | <7ng/l delta change between T=0 and T=6 reading | Negative | |

### Sample collection and preparation

Blood samples were collected on arrival at the emergency department. Upon a negative ECG, samples were sent to the laboratory for measurement of H-FABP and hsTnT on plasma.

### Biomarker analysis

Prior to analysis, aliquots were thawed on a mechanised roller for at least 20 minutes at room temperature. Once biomarker analysis had been performed, any residual sample aliquot was discarded. Biomarker analysis was only performed on samples having undergone 1 freeze thaw cycle. H-FABP (Catalogue No. FB 4025) was measured using the Randox immunoturbidimetric assay on a Cobas c701 analyzer. Highly sensitive troponin T was analysed with a Roche E170 modular analyser (Roche Diagnostics, Basel, Switzerland).

### Results

Of the 62 patients included in the study, the total number of patients with positive H-FABP at presentation was 61 and included 4 (6.45%) patients who had a troponin negative result at presentation and who were then diagnosed as AMI from 6 hour troponin measurement. These 4 patients had to wait an additional 6 hours for a second blood sample to be taken plus a further 1 hour for their troponin result to be obtained. Including the 1 hour to the first troponin result more than 8 hours lapsed before AMI was diagnosed. The additional measurement of H-FABP at presentation to the Emergency Department (ED) improves the triage of chest pain patients resulting in a decrease in bed occupancy. A further 33 patients (54%) were positive for both

H-FABP and hsTnT measured from the first patient sample at presentation. Of these, 16 patients (26% overall) had a positive hsTnT value at 6 hours indicating AMI. These patients also had a wait of greater than 8 hours for AMI diagnosis, allowing more heart cell damage to occur. The remaining 17 (27.8% overall) had a negative hsTnT at 6 hours. This cohort is more likely to have heart failure, renal failure or atrial fibrillation. These patients were admitted but not necessarily stented. They may have been admitted and sent home after 24 hours and/or returned as an outpatient for further cardiac investigations. These patients would have been investigated further due to their elevated troponin level. 24 patients (39%) were negative for hsTnT in both the 0 and 6 hour samples but were positive for H-FABP in the first blood sample taken at presentation. Clinical investigations were performed on these patients because of the elevated H-FABP level for a possible cardiovascular condition.

**Table 2 Outcome of further investigation of 24 patients diagnosed non-AMI based on negative ECG and negative cardiac troponin T at T=0 and T=6 (current pathway). These patients were H-FABP positive at T=0(16 were diagnosed with a cardiovascular condition).**

| **Investigation** | **Result** | **Outcome** | **Comment** |
|---|---|---|---|
| 8 x inpatient angio grams | 5 x ischaemic heart disease | 4 patients required stents (PCI); 1 x aortic stenosis | Patients not identified in current pathway |
| | 3 x non-ischaemic heart disease | N/A | N/A |
| 2 x outpatient angio gram | 1 x mild aortic stenosis | No follow up | |
| | 1 x normal | No follow up | |
| 2 x inpatient Exercise Stress Tests | 1 x borderline | Patient discharged for outpatient angiogram which identified triple vessel disease. Patient had coronary artery bypass | Patient not identified in current pathway. |
| | 1 x normal | Patient was discharged but then admitted STEMI/PCI | Patient not identified in current pathway |
| 2 x outpatient Exercise Stress Tests | 1 x awaiting angiogram | | Patient not identified in current pathway |
| | 1 x normal | Admitted Stroke | Patient not identified in current pathway |
| 3 x inpatient Echocardiogram (ECHO) | 3 x coronary problems | | Patients not identified in current pathway |
| 2 x outpatient dobutamine stress ECHO | 1 x booked but not done | 1 x NSTEMI & inpatient angiogram identified triple vessel disease requiring coronary artery bypass | Patients not identified in current pathway |
| | 1 x normal | | |
| 5 x discharged | 1 x unstable angina; 1 x DVT; 1 x elevated H-FABP; | | |
| | 2 x normal | | |

Of the 24 patients who were H-FABP positive and were not identified by the current pathway (ECG and cardiac troponin), 18 had a cardiovascular condition.

### Case Studies

### Example 1

A 42 year old male patient who woke from sleep at 03.30 complaining of chest discomfort radiating into the left arm. The patient's first blood sample was taken at 04.30 and the ECG showed nonspecific changes. The first troponin measurement of 14ng/l again was classified as normal. The six hour troponin measurement was positive at 38ng/l (≥7ng/l delta change). However, the H-FABP measurement on the first blood sample at arrival was 4.38ng/ml, which was above the 95^{th} percentile (3.55ng/ml) so the clinicians prioritised the patient to the cardiac ward for treatment. The patient was diagnosed as NSTEMI and had a stent placed in an occluded circumflex artery.

### Example 2

A 67 year old female who had chest pain radiating into her arms and jaw was woken from her sleep at 04.10. The first blood sample was taken at 06.30 and her ECG was normal. The medical physician then ordered her troponin to be measured and the level came back as 12ng/l which was normal. After a six hour wait the patient had another blood sample taken and troponin measured. This time the value was 18ng/l which was below the diagnostic 7ng/l increase required to diagnose MI. The patient's H-FABP at admission was 4.14ng/l indicating myocardial ischaemia. The patient had an exercise stress test which was also normal. However, in view of the clinical history and the elevated H-FABP, the patient had cardiac catheterisation which revealed right coronary artery and circumflex artery disease. A stent was placed in the right coronary artery and the circumflex disease was treated medically.

Table 2 and the Examples clearly show the utility of the method in highlighting individuals with a cardiovascular condition and who on the basis can proceed to prompt, life-saving medical intervention. Surprisingly, some individuals presenting with chest pain and who had an elevated H-FABP and negative troponin, were diagnosed with deep vein thrombosis and stroke; the method of the invention thus represents an indicator of atherosclerosis, ischemic stroke and deep vein thrombosis.

## Claims

1. A method of determining a possible cardiovascular condition in an individual with chest pain comprising determining H-FABP in an ex *vivo* sample taken from the individual who has a negative electrocardiogram and negative cardiac troponin and based on the determination of H-FABP categorising the individual as healthy or having a possible cardiovascular condition **characterised in that** a positive H-FABP determination indicates that the possible cardiovascular condition is atherosclerosis, ischemic stroke or deep vein thrombosis.

2. The method of the previous claims in which H-FABP is measured within one hour of admission to a medical facility.

3. The method of the previous claims in which the H-FABP level is greater than the 95th percentile of the upper reference level.

4. The method of the previous claims in which the sample is blood, serum or plasma.

## Patentansprüche

1. Verfahren zur Bestimmung eines möglichen kardiovaskulären Zustands bei einem Individuum mit Brustschmerzen, umfassend das Bestimmen von H-FABP in einer Ex-vivo-Probe, die dem Individuum entnommen wurde, das ein negatives Elektrokardiogramm und negatives kardiales Troponin aufweist, und basierend auf der Bestimmung von H-FABP das Einstufen des Individuums als gesund oder mit einem möglichen kardiovaskulären Zustand, **dadurch gekennzeichnet, dass** eine positive H-FABP-Bestimmung anzeigt, dass der mögliche kardiovaskuläre Zustand Atherosklerose, ischämischer Schlaganfall oder tiefe Venenthrombose ist.

2. Das Verfahren der vorhergehenden Ansprüche, bei dem H-FABP innerhalb einer Stunde nach Aufnahme in eine medizinische Einrichtung gemessen wird.

3. Verfahren nach den vorhergehenden Ansprüchen, bei dem der H-FABP-Spiegel größer als das 95. Perzentil des oberen Referenzspiegels ist.

4. Verfahren nach den vorhergehenden Ansprüchen, bei dem die Probe Blut, Serum oder Plasma ist

## Revendications

1. Procédé de détermination d'un état cardiovasculaire possible chez un individu souffrant de douleur thoracique comprenant la détermination de H-FABP dans un échantillon ex vivo prélevé sur l'individu qui a un électrocardiogramme négatif et une troponine cardiaque négative et basé sur la détermination de H-FABP catégorisant l'individu comme en bonne santé ou ayant un état cardiovasculaire possible **caractérisé en ce qu'**une détermination positive de H-FABP indique que l'état cardiovasculaire possible est l'athérosclérose, un accident vasculaire cérébral ischémique ou une thrombose veineuse profonde.

2. Procédé selon le revendication précédente, dans lequel la H-FABP est mesurée dans l'heure qui suit l'admission dans un établissement médical.

3. Procédé selon les revendications précédentes, dans lequel le niveau de H-FABP est supérieur au 95e centile du niveau de référence supérieur.

4. Procédé selon les revendications précédentes, dans lequel l'échantillon est du sang, du sérum ou du plasma.
